# EUROPÄISCHE PATENTSCHRIFT

(11) **EP 1 771 458 B1**
(45) Veröffentlichungstag und Bekanntmachung des Hinweises auf die Patenterteilung: **05.08.2009**
(21) Anmeldenummer: 05701353.4
(22) Anmeldetag: 04.02.2005
(51) Int. Cl.: C07F 9/22, C05G 3/08, C05B 15/00, A61K 31/664, A61P 1/04, A61P 13/02

(54) **N-PHENYLPHOSPHORSÄURETRIAMIDE, VERFAHREN ZU IHRER HERSTELLUNG UND DEREN VERWENDUNG ALS MITTEL ZUR REGULIERUNG BZW. HEMMUNG DER ENZYMATISCHEN HARNSTOFF-HYDROLYSE**
N-PHENYLPHOSPHORIC ACID TRIAMIDES, METHOD FOR THE PRODUCTION THEREOF AND THEIR USE AS AGENTS FOR REGULATING OR INHIBITING ENZYMATIC UREA HYDROLYSIS
TRIAMIDES D'ACIDE N-PHENYL-PHOSPHORIQUE, PROCEDES POUR LES PRODUIRE ET LEUR UTILISATION COMME AGENTS POUR REGULER OU INHIBER L'HYDROLYSE ENZYMATIQUE DE L'UREE

(30) Priorität: 23.07.2004 DE 102004035742
(43) Veröffentlichungstag der Anmeldung: 11.04.2007
(73) Patentinhaber: SKW STICKSTOFFWERKE PIESTERITZ GmbH, 06886 Lutherstadt Wittenberg (DE)
(72) Erfinder: HUCKE, André, 06898 Reinsdorf (DE); NICLAS, Hans-Joachim, 12435 Berlin (DE); WOZNIAK, Hartmut, 04451 Cunnersdorf (DE); MICHEL, Hans-Jürgen, 04827 Machern (DE); SCHUSTER, Carola, 06130 Halle/Saale (DE)
(74) Vertreter: Schneider, Michael
(86) Internationale Anmeldenummer: PCT/EP2005/001157
(87) Internationale Veröffentlichungsnummer: WO 2006/010389

(56) Entgegenhaltungen:
- EP-A- 0 119 494
- WO-A-96/40856
- US-A- 4 242 325
- US-A- 4 517 004

## Beschreibung

Die vorliegende Erfindung betrifft neue N-(2-Nitrophenyl)phosphorsäuretriamide, Verfahren zu ihrer Herstellung, diese enthaltende Zusammensetzungen und die Verwendung dieser N-(2-Nitrophenyl)phosphorsäuretriamide oder diese enthaltenden Zusammensetzungen als Mittel zur Regulierung bzw. Hemmung der enzymatischen (ureasekatalysierten) Harnstoff-Hydrolyse (auch in Kombination mit Mitteln zur Einschränkung der Nitrifikation) zur Vermeidung von Stickstoffverlusten bei der Anwendung harnstoffbasierter Düngemittel, sowie zur Reduzierung der Ammoniakbelastung in Tierställen infolge einer weitestgehenden Ausschaltung der Harnstoff-Hydrolyse und als Zusatz zu Futterharnstoff im Rahmen der Tierernährung, speziell von Wiederkäuern. Die Erfindung betrifft weiterhin Düngemittelzusammensetzungen, welche *N-*Phenylphosphorsäuretriamide und ein harnstoffbasiertes Düngemittel enthalten.

Harnstoff ist ein ursprünglich biogenes Stoffwechselprodukt, das durch das Enzym Urease in Ammoniak und Kohlendioxid gespalten wird. Die Reaktion verläuft außerordentlich schnell und effektiv und ist somit für N-Verluste bei der Anwendung von harnstoffbasierten Düngemitteln verantwortlich. Diese sind besonders hoch, wenn der Boden nicht über eine ausreichende Sorptionskraft verfügt, um das frei gewordene Ammoniak in Form von Ammoniumionen zu binden. Dadurch gehen der Landwirtschaft jährlich beträchtliche Mengen an Stickstoff verloren, die auf diese Weise zur Umweltbelastung beitragen und andererseits einen erhöhten Düngemittelbedarf erfordern.

Darüber hinaus können unter ungünstigen Klimabedingungen und/oder bei Ausbringung auf leichte Böden spontan hohe Ammoniakkonzentrationen im Boden auftreten, die dann zusätzlich die Keimung und das Auflaufen der Jungpflanzen negativ beeinträchtigen.

Da Harnstoff das Stickstoffdüngemittel mit dem prozentual größten N-Gehalt und der weltweit mit Abstand dominierende N-Dünger ist, wird die Suche nach praktikablen Lösungen zur Reduzierung der ureasebedingten N-Verluste verständlich. Um dieses Ziel zu erreichen, ist eine Vielzahl von Lösungen vorgeschlagen worden. Zu nennen sind in diesem Zusammenhang die saure Umhüllung von Harnstoffprills oder -granalien, um auf diese Weise entstehenden Ammoniak durch Salzbildung abfangen zu können oder das Coating mit Substanzen, wodurch Harnstoff verlangsamt freigesetzt wird und somit das entstehende Ammoniak problemlos »abgepuffert« werden kann.

Außerdem verursacht die ureasekatalysierte Harnstoff-Hydrolyse durch Spaltung des in Kot und vor allem in Harn befindlichen Harnstoffs in Tierställen zum Teil erhebliche Ammoniaklasten, die - abgesehen von einer Geruchsbelästigung bei entsprechend hoher Konzentration - die Entwicklung und das Wachstum der Tiere negativ beeinflussen.

Die N-Verluste aus der ureasekatalysierten Hydrolyse des Harnstoffs und der Nitrifikation können unter ungünstigen Bedingungen, besonders in tropischen und subtropischen Klimaten, bis zu 50 % betragen. Um dieses Verlustpotenzial zu minimieren, werden bedarfsgerecht mehrfach geteilte Düngergaben empfohlen, die jedoch für den Landwirt infolge zusätzlicher Applikationskosten mit erheblichen arbeitswirtschaftlichen Nachteilen und entsprechenden Mehraufwendungen verbunden sind.

Möglichkeiten zur Einschränkung der Stickstoffverluste bestehen in der gezielten Hemmung der ureasekatalysierten Harnstoff-Hydrolyse auf der einen und der Nitrifikationshemmung auf der anderen Seite. Dabei erscheint im ersten Fall der Einsatz solcher Substanzen aussichtsreich, die zu einer Ureasehemmung führen, wobei sich neben der Anwendung für Düngungszwecke selbstverständlich auch die Anwendung zur Minimierung der Ammoniakbelastung in Tierställen oder deren Zusatz zu Futterharnstoff anbietet.

Der Einsatz von Ureaseinhibitoren ist eine effektive Möglichkeit, die unter Normalbedingungen außerordentlich schnell verlaufende enzymatische Harnstoff-Hydrolyse deutlich zu verlangsamen. Durch die Verzögerung dieser Enzymreaktion kann der Düngeharnstoff unzersetzt in tiefere Bodenschichten penetrieren.

Damit sind Ammoniakverluste durch das Sorptionspotential der darüber befindlichen Bodenschichten, anders als an der Bodenoberfläche, nahezu ausgeschlossen. Außerdem gelingt es auf diesem Wege, Harnstoff und harnstoffhaltige Dünger für leichte Bodenstandorte verlustfrei zur Anwendung zu bringen.

In Tierställen kann die Emission von Ammoniak aus Dung und tierischen Exkrementen durch Zusatz eines Ureaseinhibitors auf effektive Weise eingeschränkt werden.

Aus der Sicht der verlustfreien und damit umweltentlastenden Lagerung und Ausbringung von organischen Düngern wie Mist oder Gülle ist der Einsatz von Ureaseinhibitoren, gegebenenfalls auch in Kombination mit Nitrifikationsinhibitoren, gleichfalls eine empfehlenswerte Maßnahme, um die Dünger- und damit Düngungseffizienz von Wirtschaftsdüngern zu erhöhen.

Es ist bekannt, dass speziell in der Tierfütterung von Wiederkäuern die Versorgung der Tiere mit proteinreicher und damit leistungsfördernder Nahrung teilweise ein finanzielles Problem für den Landwirt darstellt, in einigen Regionen der Erde jedoch auch aus klimatischen Gründen ein über das volle Jahr unlösbares Problem ist. Aus heutiger Sicht ist die Substitution pflanzlichen Eiweißes durch Tiermehl in der Wiederkäuerernährung gesundheitspolitisch nicht zu vertreten. Um dieser Situation gerecht zu werden, bietet sich eine teilweise Substitution der hochwertigen proteinreichen pflanzlichen Ernährung der Tiere durch sogenannte »non-protein-nitrogen-Verbindungen« (NPN-Verbindungen) an. Diese Rolle kann der Harnstoff übernehmen, wenn es gelingt, die im Pansen der Tiere ablaufende ureasekatalysierte Harnstoff-Hydrolyse so zu kontrollieren, dass die freigesetzten Ammoniakraten durch anwesende Mikroorganismen sofort zu mikrobiellem Protein verarbeitet werden und demzufolge keine toxischen Effekte auslösen können. Hier bietet sich gleichfalls der Einsatz geeigneter Ureaseinhibitoren an.

Aus der Literatur ist bekannt, dass bestimmte organische, aber auch anorganische Verbindungen die ureasekatalysierte Harnstoff-Hydrolyse zu hemmen vermögen (vgl. S. Kiss, M. Simihäian, Improving Efficiency of Urea Fertilizers by Inhibition of Soll Urease Activity, Kluwer Academic Publishers (2002)).

Mit der Entdeckung der Phosphorsäureesterdiamide (DD 122 177) sind Verbindungen gefunden worden, die äußerst effektive Ureaseinhibitoren darstellen. Ähnlich wirksam ist eine Reihe von Derivaten des Phosphorsäuretriamids einschließlich des Grundkörpers (vgl. bspw. US 4,540,428, US 4,676,822, US 4,696,693, US 4,537,614, US 4,517,004, EP 0 119 487), von denen das N-(n-Butyl)thiophosphorsäuretriamid (NBTPT) als einziger Vertreter bisher kommerzialisiert wurde (IMC AGRICO Corp., Produktbezeichnung Agrotain®).

Bei genauer Prüfung dieser Substanzen zeigt es sich, dass einige relativ hydrolyseanfällig sind, wodurch vor allem ihre Wirkungsdauer und somit ihre Anwendbarkeit erheblich eingeschränkt ist. Zum anderen sind sie teilweise nur in geringer Ausbeute oder mittels aufwendiger Herstellungsverfahren erhältlich, so dass eine vertretbare Ökonomie nicht gegeben ist. Aufgrund der Hydrolyseanfälligkeit des NBTPT und dessen Instabilität in Kombination mit harnstoffbasierten Düngemitteln kommt dieser Wirkstoff als Flüssigformulierung zum Einsatz, wobei die Formulierung unmittelbar vor der Düngerapplikation mit dem harnstoffbasierten Düngemittel gemischt wird, was äußerst unökonomisch ist. Eine gleichmäßige Wirkstoffverteilung auf den Düngergranalien kann so kaum gewährleistet werden. Darüber hinaus versagt NBTPT unter anaeroben Bedingungen im Reisanbau, also gerade dort, wo die höchsten Stickstoffverluste und Ammoniakemissionen zu verzeichnen sind, da die Bildung des Sauerstoffanalogons des NBTPT und damit des eigentlichen Ureaseinhibitors nicht oder nur langsam möglich ist (vgl. Fertilizer Research 42, 251 (1995)).

Weitere, die Anwendung der erwähnten Verbindungen beeinflussende Nachteile sind, dass sie ein anderes Migrationsverhalten als Harnstoff haben. Dadurch wird der Inhibitor vom Substrat Harnstoff getrennt, was zur Beeinträchtigung der Enzymhemmung führen kann. Möglich ist aber auch, dass ursprünglich wirksame Ureaseinhibitoren, wenn sie mit Boden in Kontakt kommen, ihre Inhibitorwirkung durch Reaktion mit Bodenbestandteilen oder Fixierung an diese verlieren.

Neben den N-Verlusten durch unkontrollierte ureasekatalysierte Hydrolyse des Harnstoffs geht Stickstoff in Form von Nitrat verloren, das durch Auswaschung oder Verlagerung in tiefere Bodenschichten der Pflanzenernährung entzogen wird. Darüber hinaus können diese N-Verluste noch erhöht werden, wenn im Verlaufe der raschen Nitrifikation von Ammoniumionen relativ große Mengen an Nitrat gebildet werden, die ihrerseits durch einsetzende Denitrifikation in molekularen Stickstoff überführt werden können und damit gleichfalls für die pflanzliche Ernährung nicht mehr verfügbar sind.

Als Nitrifikationsinhibitoren sind beispielsweise substituierte Pyrazole (DD 131 063, US 3,635,690), Triazole (DE-OS 18 04 994, US 3,697,244, US 3,701,645) aber auch Wirkstoffkombinationen auf der Basis von Pyrazolverbindungen und Dicyandiamid (DD 227 957) oder von Triazolderivaten und Dicyandiamid (WO 95/22 515) vorgeschlagen worden. Weiterhin werden in US 5,364,438 neue flüssige Stickstoffdünger beschrieben, die neben gelöstem Stickstoff in Form von Harnstoff und anderen Stickstoffnährformen auch Anteile von N-(n-Butyl)thiophosphorsäuretriamid (NBTPT) und Dicyandiamid (DCD) enthalten.

Zur umfassenden Minimierung der Stickstoffverluste bei der Anwendung von harnstoffbasierten Düngemitteln bietet sich die Anwendung von Ureaseinhibitoren in Kombination mit Nitrifikationsinhibitoren an. Untersuchungen dazu haben jedoch gezeigt, dass Urease- und Nitrifikationsinhibitoren nicht beliebig miteinander kombiniert werden können, da unter bestimmten Bedingungen sich die zusätzliche Anwendung eines Nitrifikationsinhibitors zum Ureasehemmstoff negativ auf die beabsichtigte Senkung der Ammoniakverluste auswirken kann (NBTPT/DCD: Biol. Fertil. Soils 36 129 (2002)). Dieser Befund spiegelt sich auch in den Erträgen wider, die teilweise auf dem Niveau der als Vergleich herangezogenen alleinigen Harnstoffdüngung lagen (NBTFT/Carbid: Biol. Fertil. Soils 22, 89, (1996)).

US-A-4,242,325 offenbart bestimmte Phosphorsäuretriamide als Ureaseinhibitoren. Unter anderem wird auch ein 4-Nitrophenylderivat angegeben.

In der WO 96/40856 wird die Verwendung von Ureaseinhibitoren zur Unterdrückung des Ammoniakgeruchs in sanitären Einrichtungen beschrieben. Unter anderem werden substituierte Phenylphosphorsäuretriamide als geeignet angegeben.

Der vorliegenden Erfindung lag daher die Aufgabe zugrunde, solche Ureaseinhibitoren für die Praxis zur Verfügung zu steilen, die bei der Anwendung mit Düngeharn-stoff oder anderen harnstoffbasierten Düngern in der Lage sind, die enzymkatalysierte Harnstoff-Hydrolyse auf ein solches Maß zu beschränken, dass daraus resultierende Stickstoffverluste in Form von Ammoniak nahezu auszuschließen sind bzw. die Ammoniaklast in Tierställen durch spontane Zersetzung des Harnstoffs deutlich reduziert wird. Die neuen Ureaseinhibitoren sollen ohne Wirkungseinbußen mit Nitrifikationsinhibitoren kombinierbar sein, um eine weitere Verbesserung der N-Ausnutzung in harnstoffbasierten Düngemitteln zu erreichen.

Gleichermaßen sollen diese Ureaseinhibitoren die im Pansen von Wiederkäuern ablaufende Harnstoffspaltung bei Einsatz von Harnstoff im Rahmen der Tierfütterung so verlangsamen, dass die Tiere durch sonst erfolgende Ammoniak-Intoxikationen keine Schäden erleiden und andererseits den auf diese Weise angebotenen Stickstoff für die körpereigene Proteinbiosynthese verwerten können.

Ein weiteres Anwendungsgebiet ist die Medizin. Die Ureaseinhibitoren können zur Prophylaxe oder Therapie von Störungen oder Erkrankungen eingesetzt werden, die direkt oder indirekt durch Ureaseaktivität induziert oder begünstigt werden. Beispiele dafür sind die Katheterverkrustung, entzündliche und ulzeröse Magen- und Darmerkrankungen, Urolithiasis, Pyelonephritis, Nephrolithiasis, Ammoniak-Encephalopathie, hepatische Encephalopathie, hepatisches Koma, Harnwegsinfektionen und gastrointestinale Infektionen. Diese können beispielsweise durch Urease produzierende Mikroorganismen wie Helicobacter pylori verursacht werden.

Dies Aufgaben werden erfindungsgemäß durch die Bereitstellung und Verwendung der N-(2-Nitrophenyl)phosphorsäuretriamide mit den in Anspruch 1 definierten Strukturen gelöst.

Es hat sich nämlich überraschenderweise gezeigt, dass die erfindungsgemäß bereitgestellten und eingesetzten N-(2-Nitrophenyl)phosphorsäuretriamide bei geeigneter Substitution am Phenylrest hocheffektive Ureaseinhibitoren mit außerordentlich langanhaltender Wirkung darstellen Neben einer ausreichenden Hydrolysebeständigkeit sind die erfindungsgemäßen N-(2-Nitrophenyl)phosphorsäuretriamide außerdem technisch problemlos und kostengünstig aus einfachen Ausgangsstoffen herstellbar. Die erfindungsgemäßen N-(2-Nitrophenyl)phosphorsäuretriamide sind außerdem mit gebräuchlichen Verfahren leicht in Harnstoff oder harnstoffbasierte Düngemitteln einarbeitbar, womit eine effiziente Ausbringung zusammen mit dem Düngemittel bzw. Verfütterung an Wiederkäuer möglich ist. Sie sind sowohl ausreichend wasserlöslich ais auch gut gemäßen N-(2-Nitrophenyl)phosphorsäuretrimide ist vorhersehbar war. Ein weiterer Vorteil der erfindungsgemäßen N-(2-Nitrophenyl)phosphorsäuretriamide ist deren problemlose Kombinierbarkeit mit Nitrifikationsinhibitoren.

Die Erfindung betrifft ferner Verfahren zur Herstellung der erfindungsgemäßen *N-*N-(2-Nitrophenyl)phosphorsäuretriamide wie in Anspruch 3 definiert, Zusammensetzungen, die diese N-(2-Nitrophenyl)phosphorsäuretriamide enthalten, wie in Anspruch 3 definiert, eine Düngemittelzusammensetzung wie in Anspruch 7 definiert sowie die in den Ansprüchen 10 bis 16 definierten Verwendungen.

Weitere vorteilhafte und/oder bevorzugte Ausführungsformen der Erfindung sind Gegenstand der Unteransprüche.

Die erfindungsgemäßen N-(2-Nitrophenyl)phosphorsäuretriamide als Ureaseinhibitoren besitzen die allgemeine Formel (I): 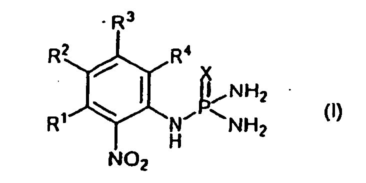 in der:
X = Sauerstoff oder Schwefel
R¹, R², R³, R⁴ = unabhängig voneinander Wasserstoff, C₁-C₈-Alkyl/Heteroalkyl, C₂-C₈-Alkenyl/Heteroalkenyl, C₂-C₈-Alkinyl/Heteroalkinyl. C₃-C₈-Cycloalkyl/Heterocycloalkyl, C₃-C₈-Cycloalkenyl/Heterocycloalkenyl, C₆-C₁₀-Aryl/C₅-C₁₀-Heteroaryl, Aralkyl, Heteroarylalkyl, Alkaryl, Alkheteroaryl, Alkoxy, Aryloxy, Hetaryloxy, Alkylthio, Arylthio, Hetarylthio, Acyl, Aroyl, Hetaroyl, Acyloxy, Aroyloxy, Hetaroyloxy, Alkoxycarbonyl, Aryloxycarbonyl, Hetaryloxycarbonyl, Alkylsulfonyl, Fluor, Chlor, Brom, Iod, Cyano, Nitro, Sulfo, Carbonyl, Carboxy, Carbamoyl, Sulfamoyl bedeuten,
wobei zwei benachbarte Reste R miteinander über eine Alkylen- bzw. Alkenylen-Kette unter Bildung eines 5-6-gliedrigen, ggf. aromatischen Ringsystems verknüpft sein können, welches ggf. ein oder mehrere Heteroatome, wie Sauerstoff, Stickstoff oder Schwefel enthalten kann,
wobei die Reste R¹-R⁴ gegebenenfalls selber und unabhängig voneinander mit einer oder mehreren der oben genannten Gruppen sowie durch Amino, Alkylamino, Dialkylamino, Hydroxy, Mercapto substituiert sein können,
sowie Salze, Tautomere und Metallkomplexe von Verbindungen der allgemeinen Formel (I), die ureaseinhibierende Wirkung haben.

Die Reste R¹-R⁴ können gegebenenfalls selber und unabhängig voneinander mit einer oder mehreren der oben genannten Gruppen sowie durch Amino, Alkylamino, Dialkylamino, Hydroxy, Mercapto substituiert sein. Zwei benachbarte Reste R (z.B. R¹ und R²) können miteinander über eine Alkylen- bzw. Alkenylen-Kette unter Bildung eines 5-6-gliedrigen, ggf. aromatischen Ringsystems verknüpft sein, welches ggf. ein oder mehrere Heteroatome, wie Sauerstoff, Stickstoff oder Schwefel enthalten und durch oben genannte Gruppen substituiert sein kann.

Ein bevorzugtes N-(2-Nitrophenyl)phosphorsäuretriamide der vorliegenden Erfindung entspricht der Formel (II):

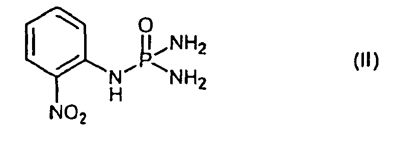

Außerdem umfasst die Erfindung Salze, Tautomere und Metallkomplexe von Verbindungen der allgemeinen Formel (I) oder (II), die ureaseinhibierende Wirkung haben.

Selbstverständlich wird der Fachmann die in der allgemeinen Formel (I) angegebenen Reste bzw. Gruppen so auswählen, dass keine unmöglichen Moleküle entstehen, z.B. chemisch oder sterisch unmögliche.

Die im folgenden erwähnten Alkyl-, Alkenyl- oder Alkinyl-Gruppen mit der entsprechenden Kohlenstoffanzahl können geradkettig oder verzweigt und einfach oder mehrfach ungesättigt sein.

Im folgenden wird zwar zur Vermeidung unnötiger Redundanz auch gelegentlich der Einfachheit halber nur der Begriff »Alkyl-Gruppe«, »Heteroalkyl-Gruppe« oder »Cycloalkyl-Gruppe« etc. verwendet, jedoch sollen jeweils die entsprechenden ungesättigten Gruppen umfaßt sein. Dem Fachmann ist klar, daß Alkenyl- oder Alkinyl-Gruppen mindestens 2 Kohlenstoffatome und cyclische Kohtenwasserstoff-Gruppen mindestens 3 Kohlenstoffatome aufweisen müssen.

Der Begriff »Alkyl« bezieht sich, und zwar in jeder Kombination mit beliebigen anderen Gruppen, insbesondere auf eine Alkyl-Gruppe, die 1 bis 8 Kohlenstoffatome aufweist, z.B. eine Methyl-, Ethyl-, n-Propyl-, Isopropyl-, n-Butyl-, Isobutyl-, tert-Butyl, Amyl, Isoamyl, n-Hexyl-, 2,2-Dimethylbutyl- oder n-Octyl-Gruppe.

Der Begriff »Alkenyl« bezieht sich, und zwar in jeder Kombination mit beliebigen anderen Gruppen, insbesondere auf eine Alkenyl-Gruppe, die 2 bis 8 Kohlenstoffatome aufweist, z.B. eine Ethenyl-, n-Propenyl-, Isopropenyl-, n-Butenyl-, Isobutenyl-, tert-Butenyl, n-Hexenyl-, 2,2-Dimethylbutenyl-, n-Octenyl-, Allyl-, Isoprenyl- oder Hex-2-enyl-Gruppe.

Der Begriff »Alkinyl« bezieht sich, und zwar in jeder Kombination mit beliebigen anderen Gruppen, insbesondere auf eine Alkinyl-Gruppe, die 2 bis 8 Kohlenstoffatome aufweist, z.B. eine Ethinyl-, n-Propinyl-, Isopropinyl-, n-Butinyl-, Isobutinyl-, tert-Butinyl, n-Hexinyl-, 2,2-Dimethylbutinyl- oder n-Octinyl-Gruppe.

Der Begriff »Heteroalkyl« bezieht sich bzgl. des Alkylteils auf eine oben definierte Alkyl-Gruppe, soll aber auch eine entsprechende Heteroalkenyl- oder Heteroalkinyl-Gruppe umfassen, in der ein oder mehrere Kohlenstoffatome durch mindestens ein Sauerstoff-, Stickstoff-, Phosphor- oder Schwefelatom ersetzt sind.

Es ist klar, daß sämtliche der oben definierten Gruppen mit sich selbst oder anderen der oben definierten Gruppen substituiert sein können, sofern die ureaseinhibierende Wirkung erhalten bleibt.

Der Begriff »Aryl« bezieht sich auf eine aromatische cyclischeGruppe, die einen oder mehrere Ringe aufweist, und durch ein Gerüst gebildet wird, das 6 bis 10 Ringkohlenstoffatome enthält. Selbstverständlich kann im Falle von mehreren Ringen einer oder auch mehrere Ringe ganz oder teilweise hydriert sein (ein Beispiel dafür ist die 1,2,3,4,-Tetrahydro-naphthalen-1-yl-Gruppe). Außerdem kann eine Aryl-Gruppe durch Alkyl- oder Heteroalkyl-Gruppen (jeweils wie oben definiert) substituiert sein. Beispiele sind eine Phenyl-, Naphthyl-, Inden-, 2-, 3- oder 4-Methoxyphenyl-, 2-, 3-oder 4-Ethoxyphenyl-, 4-Carboxyphenylalkyl- oder 4-Hydroxyphenyl-Gruppe.

Die Begriffe »Aralkyl« bzw. »Heteroarylalkyl« beziehen sich auf Gruppen, die entsprechend den obigen bzw. folgenden Definitionen sowohl Aryl- bzw. Heteroaryl-(weiter unten definiert) wie auch Alkyl- und/oder Heteroalkyl- (auch die entsprechenden Alkylen/Heteroalkylen- und Alkinyl/Heteroalkinyl-Gruppen) und/oder carbocyclische Gruppen (weiter unten definiert) und/oder Heterocycloalkyl-Ringsysteme (weiter unten definiert) umfassen, z.B. eine Tetrahydroisochinolinyl-, Benzyl-, 2- oder 3-Ethylindolyl- oder 4-Methylpyridino-Gruppe.

Die Begriffe »Aralkyl« bzw. »Heteroarylalkyl« sollen, zur Vermeidung unnötiger Redundanz, auch die Begriffe »Alkaryl« bzw. »Alkheteroaryl« umfassen.

Der Begriff »Cycloalkyl« bzw. »carbocyclisch« bezieht sich auf eine gesättigte oder teilweise ungesättigte, cyclische, ggf. verzweigte, Gruppe, die einen oder mehrere Ringe aufweist, die ein Gerüst bilden, welches 3 bis 8 Kohlenstoffatome enthält, z.B. eine Cyclopropyl-, Cyclobutyl-, Cyclopentyl-, Cyclohexyl-, Tetralin-, Cyclopentenyl-oder Cyclohex-2-enyl-Gruppe.

Der Begriff »Heterocycloalkyl« bezieht sich auf die oben definierten Cycloalkyl- bzw. carbocylischen Gruppen, bei denen ein oder mehrere Kohlenstoffatome durch ein oder mehrere Sauerstoff-, Stickstoff-, Phosphor- oder Schwefelatome ersetzt sind. Konkrete Beispiele sind Aziridin-, Furan-, Pyrrolidin-, Piperidin-, Morpholin-, Oxazolidin-, Thiazolidin-, N-Methylpiperazino oder N-Phenylpiperazin-Gruppen.

Der Begriff »Heteroaryl« bezieht sich auf eine Aryl-Gruppe mit 5 bis 10 Ringatomen, in der ein oder mehrere Kohlenstoffatome durch ein Sauerstoff-, Stickstoff-, Phosphor- oder Schwefelatom ersetzt sind. Beispiele sind Pyrrol-, Furan-, Thiophen-, Pyrazol-, Isoxazol-, Isothiazol-, Imidazol-, Oxazol-, Thiazol-, 1,2,4-Triazol-, 1,2,4-Oxadiazol-, 1,2,4-Thiadiazol-, 1,3,4-Oxadiazol-, 1,3,4-Thiadiazol-, 1,2,5-Oxadiazol-, 1,2,5-Thiadiazol, Tetrazol, Pyridin-, Pyridazin-, Pyrimidin-, Pyrazin-, 1,2,3-Triazin-, 1,2,4-Triazin-, 1,3,5-Triazin- und Indol-Gruppen.

Es sei nochmal darauf hingewiesen, daß sämtliche der oben definierten Gruppen sowohl mit sich selbst als auch mit anderen der oben definierten Gruppen substituiert sein können, sofern die ureaseinhibierende Wirkung erhalten bleibt

Die erfindungsgemäßen Verbindungen I sind durch analoge Anwendung bekannter Verfahren zugänglich (Chem. Ber. 26, 2937 (1893); J. Chem. Soc. 81, 1362 (1902); Z. Obsc. Chim. 30, 4048 (1960)), wobei X, R¹ - R⁵ die oben beschriebene Bedeutung besitzen. Vorzugsweise erfolgt die Herstellung der N-(2-Nitrophenyl)phosphorsäuretriamide in der Weise, dass man
a₁) 2-Nitroaniline oder deren Hydrochloride mit Phosphorylchlorid (POCl₃) oder Thiophosphorylchlorid (PSCl₃), gegebenenfalls in Anwesenheit eines organischen Lösungsmittels und einer tertiären Base, bei Temperaturen von 0 bis 150 °C, gegebenenfalls unter Schutzgasatmosphäre, entsprechend Gleichung (1) zu *N*-(2-Nitrophenyl)phosphorsäureamiddichloriden des Typs (A) umsetzt, wobei Verbindungen (A) mit X = S alternativ auch durch Schwefelung der entsprechenden Sauerstoffderivate erhalten werden können, 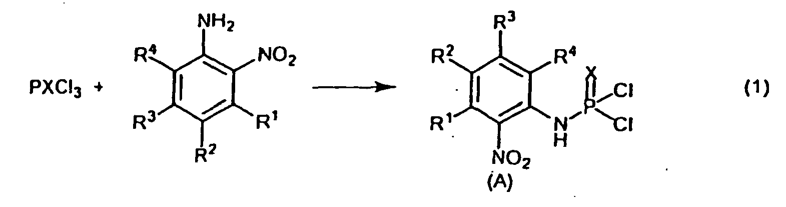 oder
a₂) Phosphorpentachlorid (PCl₅) mit einem 2-Nitroanilin, in annähernd äquimolarem Verhältnis, gegebenenfalls in einem inerten organischen Lösemittel und gegebenenfalls unter Schutzgasatmosphäre bei 0 bis 150°C entsprechend Gleichung (2) zu Verbindungen des Typs (B) reagieren lässt, welche, gegebenenfalls ohne weitere Isolation, mit einer annähernd äquimolaren Menge Ameisensäure oder Wasser zu *N-*(2-Nitrophenyl)phosphorsäureamiddichloriden des Typs (A) umgesetzt werden, wobei Verbindungen (A) mit X = S durch Schwefelung der entsprechenden Sauerstoffderivate erhalten werden können, 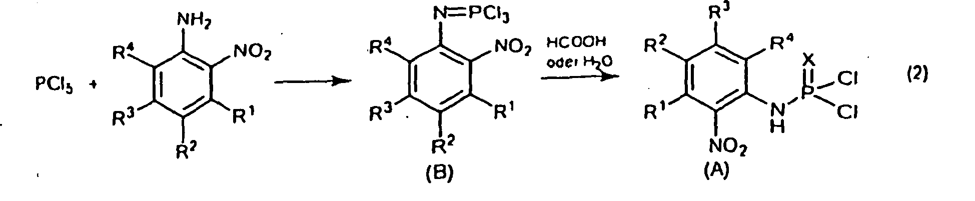 und anschließend
b) die in Stufe a₁) oder a₂) gebildeten Verbindungen des Typs (A) mit Ammoniak, gegebenenfalls in einem inerten organischen Lösemittel, bei Temperaturen von -80 bis 30 °C entsprechend Gleichung (3) zum gewünschten Endprodukt reagieren lässt:

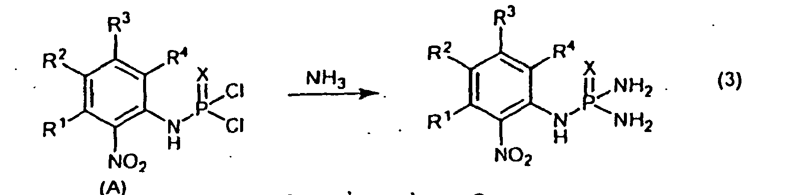

Die erfindungsgemäßen N-(2-Nitrophenyl)phosphorsäuretriamide oder diese enthaltende Zusammensetzungen weisen bei geeigneter Substitution am Phenylrest eine für praktische Belange hervorragende Inhibitionswirkung auf, wodurch sie die enzymatische Harnstoff-Hydrolyse so verlangsamen oder vorübergehend ausschalten können, dass Ammoniakverluste im Rahmen von Düngungsmaßnahmen unter Verwendung organischer und/oder mineralischer harnstoffhaltiger Stickstoffdünger auf ein Minimum reduziert werden bzw. das Auftreten schädlicher oder lästiger Ammoniakkonzentrationen in der Tierhaltung ausgeschlossen wird, beispielsweise durch Harnstoffabbau in tierischen Exkrementen oder durch den Abbau von Futterharnstoff im Pansen im Rahmen der Wiederkäuerernährung.

Dabei ist es unerheblich, ob sich die Wirkung der erfindungsgemäßen *N-*N-(2-Nitrophenyl)phosphorsäuretriamide oder diese enthaltenden Zusammensetzungen auf Düngungsmaßnahmen oder auf vorbeugend Maßnahmen zur Vermeidung hoher Ammoniakkonzentrationen in Tierställen erstreckt oder aber für den Einsatz von Futterharnstoff im Rahmen der Wiederkäuerernährung genutzt wird.

Die erfindungsgemäßen Verbindungen werden vorzugsweise gemeinsam mit harnstoffbasierten Düngemitteln, vorzugsweise in einer Menge von 0,001 bis 10 Gew.-%, bezogen auf das Gewicht des harnstoffbasierten Düngemittels, ausgebracht oder dem Futterharnstoff bzw. den in Tierställen anfallenden tierischen Ausscheidungen zugesetzt. Dabei ist es beispielsweise im Rahmen von Düngungsmaßnahmen unwesentlich, ob sie oberflächig vorher auf den Dünger aufgebracht, darin inkorporiert oder gemeinsam oder in vertretbarem Zeitraum getrennt von harnstoffbaltigen Düngemitteln ausgebracht werden.

Ein weiterer Gegenstand der Erfindung sind somit Zusammensetzungen, die die er-findungsgemäß eingesetzten N-(2-Nitrophenyl)phosphorsäuretriamide und ein harnstoffbasiertes mineralisches und/oder organisches Düngemittel enthalten.

Die erfindungsgemäß vorgeschlagenen Verbindungen können zur gleichzeitigen Verhinderung bzw. Einschränkung der ureasekatalysierten Harnstoff-Hydrolyse sowie der Nitrifikation auf die oben beschriebene Weise mit einer oder mehreren der folgenden Verbindungen, bei denen es sich um Nitrifikationsinhibitoren handelt, beispielsweise in einer Menge von 0,01 bis 10 Gew.-%, bezogen auf das Gewicht des harnstoffbasierten Düngemittels, kombiniert werden:
a) Pyrazol-Derivate der allgemeinen Formel (IV), oder Salze oder Komplexverbindungen davon: 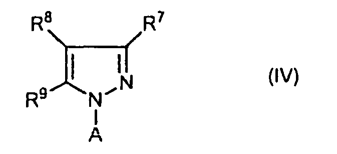 in der
   R⁷, R⁸, R⁹ unabhängig voneinander Wasserstoff, Halogen, C₁-C₈-Alkyl, oder C₃-C₈-Cycloalkyl
      und
   A den Rest H
      oder den Rest 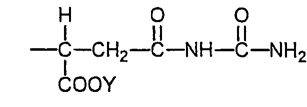 mit Y = H, Na, K, NH₄
      oder den Rest
      -CH₂-B mit B = (Di)alkylamino,
      oder den Rest 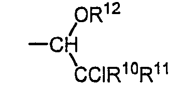 mit R¹⁰, R¹¹ = H oder Cl und
   R¹² = H oder 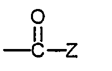 mit Z = C₁-C₈-Alkoxy, C₁-C₈-Alkylamino, C₆-C₁₀-Arylamino oder den Rest 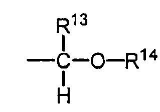 mit R¹³ , R¹⁴ = H, C₁-C₈-Alkyl, C₇-C₁₈-Alkylaryl, C₆-C₁₀-Aryl oder die Reste 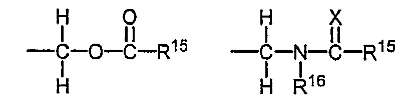 mit R¹⁵ = C₁-C₂₀-Alkyl, C₃-C₈-Cycloalkyl, C₆-C₁₀-Aryl oder Alkylaryl aus C₁-C₄-Alkyl- und C₆-C₁₀-Arylgruppen; mit X = Sauerstoff oder Schwefel sowie mit R¹⁶ = C₁-C₄-Alkyl, C₃-C₈-Cycloalkyl, C₆-C₁₀-Aryl oder H,
   bedeuten und die aufgeführten Alkyl- und Arylreste mit sich selber sowie durch C₁-C₄-Alkylsulfonyl, C₁-C₄-Alkoxy, C₁-C₄-Acyl, Halogen, Hydroxyl, Trimethylsilyl, Amino, Nitro, Cyano, Carbonyl, Carboxyl oder C₁-C₅-Carboxyalkyl substituiert sein können,
b) 1H-1,2,4-Triazole, oder deren Salze oder Komplexverbindungen,
c) Dicyandiamid.

Zur Verwendung als Ureaseinhibitoren, beispielsweise zur Senkung der Stickstoffverluste bei der Düngung mit Düngeharnstoff oder harnstoffbasierten Düngern oder zur Verminderung der Ammoniakbelastung aus dem Dung bzw. den tierischen Exkrementen in Tierställen oder zur Vermeidung toxischer Effekte beim Einsatz von Futterharnstoff können die erfindungsgemäß geeigneten Verbindungen auf verschiedene Art und Weise ausgebracht bzw. aufgebracht werden.

Die erfindungsgemäßen Verbindungen können in den Harnstoff oder in harnstoffhaltige Düngemittel vor oder während der Granulation aus der schmelzflüssigen Phase eingearbeitet werden. Weiterhin können sie auf die Oberfläche der Harnstoff- bzw. Düngemittelgranalien aufgebracht oder flüssigen harnstoffhaltigen Düngemitteln zugesetzt werden. Schließlich ist die Zugabe der Ureaseinhibitoren zu organischen harnstoffhaltigen Düngern wie Dung oder Gülle möglich. Darüber hinaus können die erfindungsgemäßen Verbindungen auch zusätzlich zur Ausbringung harnstoffbasierter Düngemittel in einem vor- oder nachgelagerten Arbeitsschritt separat auf dem Feld ausgebracht werden. Die erfindungsgemäßen Verbindungen können dabei in reiner Form als pulverfömiger Stoff, Granulat oder Schmelze, wässrige Lösung oder als spezielle Formulierung, versetzt mit den üblichen und dem Fachmann bekannten Hilfs-, Träger- und Streckstoffen oder einer Kombination dieser Mittel, eingesetzt werden. Dabei ist es unerheblich, ob der wirksame Inhaltsstoff in flüssiger Form als z. B. Lösung, Emulsion oder Supension oder in fester Form als stäub- oder dispergierbares Pulver formuliert wird. Benetzbare Pulver, emulgierbare Konzentrate und Suspensionskonzentrate enthalten gewöhnlich, aber nicht notwendigerweise, oberflächenaktive Mittel, z. B. ein Benetzungs-, Dispersions-, Emulgierungs oder Suspensionsmittel. Die jeweiligen Formulierungsverfahren entsprechen dem Stand der Technik und sind dem Fachmann bekannt.

Die erfindungsgemäßen Verbindungen, Zusammensetzungen und Düngemittel können beispielsweise bei der sowie zur Fertigation verwendet werden. Unter Fertigation versteht man die gezielte Zufuhr an Nährstoffen mit dem Bewässerungswasser, das z.B. durch Tropfbewässerung, Besprühen oder Berieseln ausgebracht werden kann. Die Pflanzen erhalten nur die zum optimalen Wachstum erforderliche Menge an Wasser, so dass kein Überschußwasser entsteht. Mangels einer vertikalen Wasserbewegung im Boden unterhalb der Durchwurzelungszone treten Nährstoffauswaschungsverluste praktisch nicht auf. Die Tropfbewässerung, Besprühung oder Berieselung mit Ureaeinhibitoren kann beispielsweise nach der Düngung erfolgen oder auch gemeinsam mit der Düngung. Natürlich besteht keine Beschränkung auf wässrige Lösungen oder sonstige Formulierungen. Beipielsweise können auch versprühbare Suspensionen feiner Teilchen verwendet werden. Insofern sei beispielhaft auf EP 1 378 499 und WO2004/013253 verwiesen.

Die vorliegende Erfindung soll nun anhand der folgenden Beispiele ohne Beschränkung und somit jediglich zur Veranschaulichung erläutert werden.

### Beispiele

### Beispiel 1

### N-(2-Nitrophenyl)phosphorsäuretriamid

In einem 100 ml Kolben mit Rückflusskühler und Trockenrohr werden 4,14 g (0,03 mol) 2-Nitroanilin und 6,25 g (0,03 mol) Phosphorpentachlorid in 50 ml Toluol suspendiert und unter Rühren 4 h zum Sieden erhitzt. Nach Abkühlen auf 80 °C werden langsam 1,38 g (0,03 mol) Ameisensäure zugegeben. Man lässt auf Raumtemperatur abkühlen, zieht das Lösungsmittel im Vakuum ab und wäscht den Rückstand mit Petrolether. Das zurückbleibende Öl wird ohne weitere Reinigung in 50 ml Chloroform aufgenommen und unter Feuchtigkeitsausschluss bei -50 bis -30 °C unter Rühren zu einer Lösung von ca. 30 ml flüssigem Ammoniak in 50 ml Chloroform getropft. Anschließend lässt man über Nacht bei Raumtemperatur das überschüssige Ammoniak abdampfen. Das ammoniumchloridhaltige Rohprodukt wird abgesaugt und zur Entfernung des Ammoniumchlorids entweder mit Diethylamin in Chloroform ausgekocht oder mit wenig Wasser gewaschen. Man erhält 3,6 g (55 %) *N-*(2-Nitrophenyl)phosphorsäuretriamid
Schmelzpunkt: ca. 200 °C (Zers.)
¹H-NMR (DMSO-d₆): δ [ppm] = 4,54 (s, 4 H, NH₂); 6,96 (t, 1 H, CH); 7,60 (t, 1 H, CH); 7,93 (d, 1 H, CH); 8,11 (d, 1 H, CH): 8,34 (d, 1 H, NH)
¹³C-NMR (DMSO-d₆): δ [ppm] = 118,8; 119,9 (d); 125,6; 133,9 (d); 135,6; 140,7 (d) ³¹P-NMR (DMSO-d₆): δ [ppm] = 8,8

### Vergleichsbeispiel 1

### N-(3-Methylphenyl)phosphorsäuretriamid

In einem 100 ml Kolben mit Rückflusskühler und Trockenrohr werden 14,3 g (0,1 mol) o-Toluidin-Hydrochlorid und 15,3 g (0,1 mol) Phosphorylchlorid in 50 ml Toluol suspendiert und unter Rühren 4 h zum Sieden erhitzt. Nach Abkühlen wird das Lösungsmittel von der entstandenen Lösung im Vakuum abgezogen und der Rückstand mit Petrolether gewaschen. Das zurückbleibende Cl wird ohne weitere Reinigung in 50 ml Chloroform aufgenommen und unter Feuchtigkeitsausschluss bei -50 bis -30 °C unter Rühren zu einer Lösung von ca. 70 ml flüssigem Ammoniak in 100 ml Chloroform getropft. Anschließend lässt man über Nacht bei Raumtemperatur das überschüssige Ammoniak abdampfen. Das ammoniumchloridhaltige Rohprodukt wird abgesaugt und zur Entfernung des Ammoniumchlorids entweder mit Diethylamin in Chloroform ausgekocht oder mit wenig Wasser gewaschen. Man erhält 9,1 g (49 %) *N*-(3-Methylphenyl)phosphorsäuretriamid.
Schmelzpunkt: 159 - 162°C
¹H-NMR (DMSO-d₆): δ [ppm] = 2,18 (s, 3 H, CH₃); 3,9 (d, 4 H, NH₂); 6,52 (d, 1 H, NH); 6,81 (d, 1 H, CH); 6,87-6,99 (m, 3H, CH)
¹³C-NMR (DMSO-d₆) δ [ppm] = 21,3 (CH₃); 114,3 (d); 117,5 (d); 119,4; 128,2 (d); 137,3; 143,4
³¹P-NMR (DMSO-d₆): δ [ppm] = 11,6 (m)

### Beispiel 3

### N-(4-Methyl-2-nitrophenyl)phosphorsäuretriamid

In einem 200 ml Kolben mit Rückflusskühler und Trockenrohr werden 11,3 g (0,06 mol) 4-Methyl-2-nitroanilin-Hydrochlorid in 100 ml Phosphorylchlorid suspendiert und unter Rühren 4 h zum Sieden erhitzt. Anschließend verfährt man analog zu Beispiel 2. Man erhält 4,9 g (35 %) *N-*(4-Methyl-2-nitrophenyl)phosphorsäuretriamid.
Schmelzpunkt: > 180 °C (Zers.)
¹H-NMR (DMSO-d₆): δ [ppm] = 2,28 (s, 3 H, CH₃); 4,50 (d, 4 H, NH₂); 7,43 (d, 1 H, CH); 7,84 (d, 1 H, CH); 7,91 (s, 1 H, CH); 8,25 (d, 1 H, NH)
¹³C-NMR (DMSO-d₆): δ [ppm] = 19,2 (CH₃); 119,5; 124,3; 127,8; 133,2 (d); 136,5; 138,2 (d)
³¹P-NMR (DMSO-d₆): δ [ppm] = 8,9

### Beispiel 4

### Prüfung auf ureasehemmende Wirkung

30 g auf 40 % der maximalen Wasserkapazität eingestellter Boden werden mit 1 ml Harnstofflösung entsprechend 50 mg Harnstoff versetzt. Gleichzeitig erfolgt die Applikation des Wirkstoffes, vorzugsweise in der Harnstofflösung gelöst. Die Konzentrationsangaben für die einzelnen geprüften Wirkstoffe in der nachfolgenden Tabelle beziehen sich auf die im Test verwendete Menge an Carbamidstickstoff. Der Boden, auf dessen Oberfläche die Harnstofflösung (mit und ohne Wirkstoff) aufgebracht wird, befindet sich in einem luftdicht verschlossenen Gefäß, in das gleichzeitig eine Vorlage eingebracht wird, die den aus dem Harnstoff freigesetzte Ammoniak ais Ammonium auffängt. Durch tägliches Überspülen der Vorlage und Analyse der enthaltenen NH₄-N-Mengen wird die NH₃-N-Freisetzung aus dem Harnstoff bestimmt.

Aus der Summation der Ammonium-Mengen in der Vorlage wird die prozentuale Hemmung der Harnstoff-Hydrolyse in Abhängigkeit von der Zeit berechnet bzw. aus diesen Werten der t₅₀-Wert rechnerisch ermittelt.

Als t₅₀-Wert wird der Zeitpunkt in Tagen nach Versuchsbeginn verstanden, zu dem die Hemmung der Harnstoff-Hydrolyse noch 50 % beträgt.

Tabelle 1 gibt einen Überblick über die nach dieser Methode ermittelten Hemmdaten einiger ausgewählter erfindungsgemäßer Verbindungen.

**Tabelle 1: Ureasehemmung (t₅₀-Wert) nach Tagen durch N-Phenylphosphorsäuretriamide der allgemeinen Formel (I)**

| | | | |
|---|---|---|---|
| 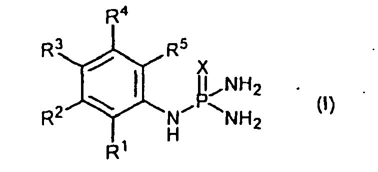 | | | |

| Verbindung Nr. | | Konz.^{a)} %HS-N-bez. | t₅₀-Wert (d) |
|---|---|---|---|
| 1 | R¹= NO₂; R², R³, R⁴, R⁵ = H | 0,05 | >25^{b)} |
| 2* | R² = CH₃; R¹, R³, R⁴, R⁵ = H | 0,5 | 11,2 |

| | | | |
|---|---|---|---|
| ^{a)} Konz. % HS-N-bez.: Konzentration in Prozent bezogen auf die verwendete Menge an Harnstoffstickstoff ^{b)} nach 25 Tagen noch 70 % Hemmung *Vergleichsbeispiel | | | |

## Patentansprüche

1. *N-*(2-Nitrophenyl)phosphorsäuretriamide der allgemeinen Formel (I): in der:
X = Sauerstoff oder Schwefel
R¹, R², R³, R⁴ = unabhängig voneinander Wasserstoff, C₁-C₈-Alkyl/Heteroalkyl, C₂-C₈-Alkenyl/Heteroalkenyl, C₂-C₈-Alkinyl/Heteroalkinyl, C₃-C₈-Cycloalkyl/Heterocycloalkyl, C₃-C₈-Cycloalkenyl/Heterocycloalkenyl, C₆-C₁₀-Aryl/C₅-C₁₀-Heteroaryl, Aralkyl, Heteroarylalkyl, Alkaryl, Alkheteroaryl, Alkoxy, Aryloxy, Hetaryloxy, Alkylthio, Arylthio, Hetarylthio, Acyl, Aroyl, Hetaroyl, Acyloxy, Aroyloxy, Hetaroyloxy, Alkoxycarbonyl, Aryloxycarbonyl, Hetaryloxycarbonyl, Alkylsulfonyl, Fluor, Chlor, Brom, Iod, Cyano, Nitro, Sulfo, Carbonyl, Carboxy, Carbamoyl, Sulfamoyl bedeuten,
wobei zwei benachbarte Reste R miteinander über eine Alkylen- bzw. Alkenylen-Kette unter Bildung eines 5-6-gliedrigen, ggf. aromatischen Ringsystems verknüpft sein können, welches ggf. ein oder mehrere Heteroatome, wie Sauerstoff, Stickstoff oder Schwefel enthalten kann,
wobei die Reste R¹-R⁴ gegebenenfalls selber und unabhängig voneinander mit einer oder mehreren der oben genannten Gruppen sowie durch Amino, Alkylamino, Dialkylamino, Hydroxy, Mercapto substituiert sein können,
sowie Salze, Tautomere und Metallkomplexe von Verbindungen der allgemeinen Formel (I), die ureaseinhibierende Wirkung haben.

2. *N-*(2-Nitrophenyl)phosphorsäuretriamid nach Anspruch 1, wobei in Formel (I) X =O und R¹=R²=R³=R⁴=H ist.

3. Verfahren zur Herstellung der *N-*(2-Nitrophenyl)phosphorsäuretriamide nach einem der Ansprüche 1 oder 2, **dadurch gekennzeichnet, dass** man
a₁) 2-Nitroaniline oder deren Hydrochloride mit Phosphorylchlorid (POCl₃) oder Thiophosphorylchlorid (PSCl₃), gegebenenfalls in Anwesenheit eines organischen Lösungsmittels und einer tertiären Base, bei Temperaturen von 0 bis 150 °C, gegebenenfalls unter Schutzgasatmosphäre, entsprechend Gleichung (1) zu *N-*(2-Nitrophenyl)phosphorsäureamiddichloriden des Typs (A) umsetzt, wobei Verbindungen (A) mit X = S alternativ auch durch Schwefelung der entsprechenden Sauerstoffderivate erhalten werden können, 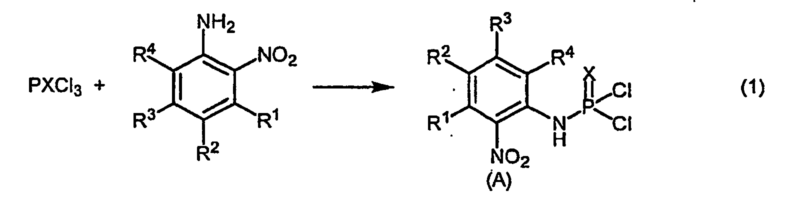 oder.
a₂) Phosphorpentachlorid (PCl₅) mit einem 2-Nitroanilin, in annähernd äquimolarem Verhältnis, gegebenenfalls in einem inerten organischen Lösemittel und gegebenenfalls unter Schutzgasatmosphäre bei 0 bis 150°C entsprechend Gleichung (2) zu Verbindungen des Typs (B) reagieren lässt, welche, gegebenenfalls ohne weitere Isolation, mit einer annähernd äquimolaren Menge Ameisensäure oder Wasser zu *N-*(2-Nitrophenyl)phosphorsäureamiddichloriden des Typs (A) umgesetzt werden, wobei Verbindungen (A) mit X = S durch Schwefelung der entsprechenden Sauerstoffderivate erhalten werden können, 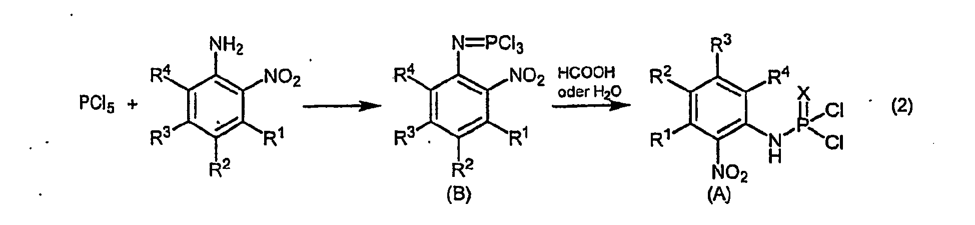 und anschließend
b) die in Stufe a₁) oder a₂) gebildeten Verbindungen des Typs (A) mit Ammoniak, gegebenenfalls in einem inerten organischen Lösemittel, bei Temperaturen von -80 bis 30 °C entsprechend Gleichung (3) zum gewünschten Endprodukt reagieren lässt:

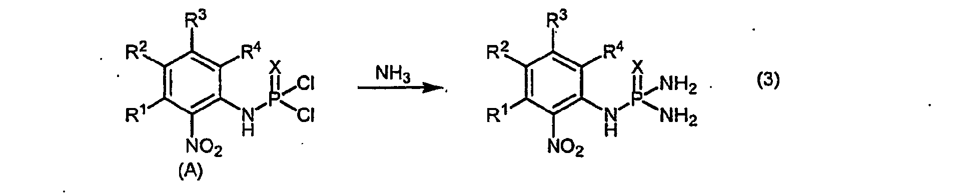

4. Zusammensetzung, **dadurch gekennzeichnet, dass** sie mindestens ein *N-*(2-Nitrophenyl)phosphorsäuretriamid nach einem der Ansprüche 1 oder 2 in einer zur Ureaseinhibition ausreichenden Menge sowie gegebenenfalls landwirtschaftlich und/oder physiologisch akzeptable bzw. verträgliche oder erwünschte . Träger-, Streck- bzw. Verdünnungsmittel, Hilfsstoffe und gegebenenfalls weitere Wirkstoffe enthält.

5. Zusammensetzung nach Anspruch 4, **dadurch gekennzeichnet, dass** als weiterer Wirkstoff mindestens ein Nitrifikationsinhibitor in einer zur Nitrifikationsinhibition wirksamen Menge enthalten ist.

6. Zusammensetzung nach Anspruch 5, **dadurch gekennzeichnet, dass** der Nitrifikationsinhibitor unter einer oder mehreren der folgenden Verbindungen ausgewählt ist:
a) Pyrazol-Derivate der allgemeinen Formel (IV), oder Salze oder Komplexverbindungen davon: 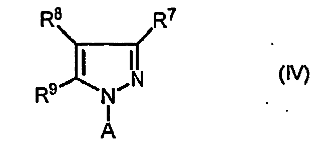 in der
R⁷, R⁸, R⁹ unabhängig voneinander Wasserstoff, Halogen, C₁-C₈-Alkyl, oder C₃-C₈-Cycloalkyl
und
A den Rest H
oder den Rest 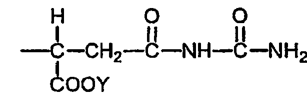 mit Y = H, Na, K, NH₄
oder den Rest
-CH₂-B mit B = (Di)alkylamino, 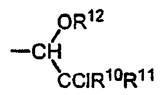 oder den Rest
mit R¹⁰, R¹¹ = H
oder Cl und 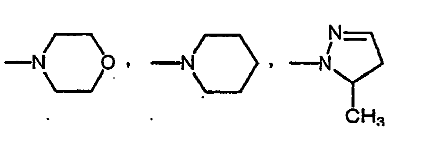
R¹² = H oder 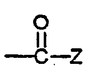 mit Z = C₁-C₈-Alkoxy, C₁-C₈-Alkylamino, C₆-C₁₀-Arylamino
oder den Rest 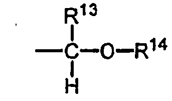 mit R¹³, R¹⁴ = H, C₁-C₈-Alkyl, C₇-C₁₈-Alkylaryl, C₆-C₁₀-Aryl
oder die Reste 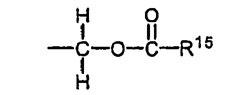 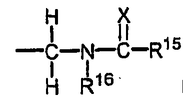 mit R¹⁵ = C₁-C₂₀-Alkyl, C₃-C₈-Cycloalkyl,
C₆-C₁₀-Aryl oder Alkylaryl aus C₁-C₄-Alkyl- und C₆-C₁₀-Arylgruppen; mit X = Sauerstoff oder Schwefel sowie mit R¹⁶ = C₁-C₄-Alkyl, C₃-C₈-Cycloalkyl, C₆-C₁₀-Aryl oder H,
bedeuten und die aufgeführten Alkyl- und Arylreste mit sich selber sowie durch C₁-C₄-Alkylsulfonyl, C₁-C₄-Alkoxy, C₁-C₄-Acyl, Halogen, Hydroxyl, Trimethylsilyl, Amino, Nitro, Sulfo, Cyano, Carbonyl, Carboxyl oder C₁-C₅-Carboxyalkyl substituiert sein können,
b) 1H-1,2,4-Triazole, oder deren Salze oder Komplexverbindungen,
c) Dicyandiamid.

7. Düngemittelzusammensetzung, **dadurch gekennzeichnet, dass** sie mindestens ein harnstoffbasiertes mineralisches und/oder organisches Düngemittel sowie mindestens ein *N-*(2-Nitrophenyl)phosphorsäuretriamid nach einem der Ansprüche 1 oder 2 und/oder eine Zusammensetzung nach einem der Ansprüche 4 bis 6 oder mindestens ein *N-*(2-Nitrophenyl)phosphorsäuretriamid nach einem der Ansprüche 1 oder 2 und mindestens einen in Anspruch 6 definierten Nitrifikationsinhibitor jeweils in einer zur Ureaseinhibition bzw. Nitrifikationsinhibition ausreichenden Menge enthält.

8. Düngemittelzusammensetzung nach Anspruch 7, **dadurch gekennzeichnet, dass** mindestens ein *N-*(2-Nitrophenyl)phosphorsäuretriamid nach einem der Ansprüche 1 oder 2 in einer Menge von 0,001 bis 10 Gew.-%, bezogen auf das Gewicht des harnstoffbasierten Düngemittels, enthalten ist.

9. Düngemittelzusammensetzung nach Anspruch 7, **dadurch gekennzeichnet, dass** mindestens einer der in Anspruch 6 definierten Nitrifikationsinhibitoren in einer Menge von 0,01 bis 10 Gew.-%, bezogen auf das Gewicht des harnstoffbasierten Düngemittels, enthalten ist.

10. Verwendung der *N-*(2-Nitrophenyl)phosphorsäuretriamide nach einem der Ansprüche 1 oder 2 oder der Zusammensetzungen nach einem der Ansprüche 4 bis 6 zur extrakorporalen Regulierung bzw. Hemmung der ureasekatalysierten Harnstoffhydrolyse.

11. Verwendung der *N-*(2-Nitrophenyl)phosphorsäuretriamide nach einem der Ansprüche 1 oder 2 oder der Zusammensetzungen nach einem der Ansprüche 4 bis 6 zur Senkung der Stickstoffverluste bei der Düngung mit Düngeharnstoff oder harnstoffbasierten Düngern.

12. Verwendung der *N-*(2-Nitrophenyl)phosphorsäuretriamide nach einem der Ansprüche 1 oder 2 oder der Zusammensetzungen nach einem der Ansprüche 4 bis 6 zur Verminderung der Ammoniakbelastung aus dem Dung bzw. den tierischen Exkrementen in Tierställen.

13. Verwendung der *N-*(2-Nitrophenyl)phosphorsäuretriamide nach einem der Ansprüche 1 oder 2 oder der Zusammensetzungen nach einem der Ansprüche 4 bis 6 zur Vermeidung toxischer Effekte bei der Verfütterung von Futterharnstoff im Rahmen der Tierernährung.

14. Verwendung der *N-*(2-Nitrophenyl)phosphorsäuretriamide nach einem der Ansprüche 1 oder 2 oder deren wässrigen Lösungen oder Flüssigformulierungen zur Stabilisierung von bereits ausgebrachten oder noch auszubringenden harnstoffbasierten Düngemitteln durch nach- oder vorgelagerte Anwendung.

15. Verwendung der Zusammensetzungen nach einem der Ansprüche 4 bis 6 zur Fertigation.

16. Verwendung der *N-*(2-Nitrophenyl)phosphorsäuretriamide nach einem der Ansprüche 1 oder 2 oder der Zusammensetzung nach Anspruch 4 zur Herstellung eines Arzneimittels zur Prophylaxe oder Therapie von Störungen oder Erkrankungen, die direkt oder indirekt durch Ureaseaktivität induziert oder begünstigt werden.

17. Verwendung nach Anspruch 16, wobei die Störung oder Erkrankung unter Katheterverkrustung, entzündlichen und ulzerösen Magen- und Darmerkrankungen, Urolithiasis, Pyelonephritis, Nephrolithiasis, Ammoniak-Encephalopathie, hepatischer Encephalopathie, hepatischem Koma, Harnwegsinfektionen und gastrointestinalen Infektionen ausgewählt ist.

18. Verwendung nach Anspruch 17, wobei die gastrointestinale Infektion durch Helicobacter pylori erfolgt.

## Claims

1. *N-*(2-nitrophenyl)phosphoric acid triamides with the general formula (I): 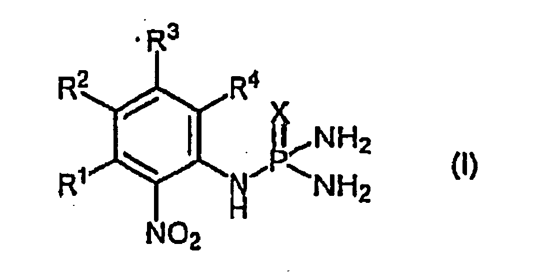 wherein:
X = oxygen or sulphur
R¹, R², R³, R⁴ = independently of one another, mean hydrogen, C₁-C₈-alkyl/heteroalkyl, C₂-C₈-alkenyl/heteroalkenyl, C₂-C₈-alkinyl/heteroalkinyl, C₃-C₈-cycloalkyl/heterocycloalkyl, C₃-C₈-cycloalkenyl/heterocycloalkenyl, C₆-C₁₀-aryl/C₅-C₁₀-heterdaryl, aralkyl, heteroarylalkyl, alkaryl, alkheteroaryl, alkoxy, aryloxy, hetaryloxy, alkylthio, arylthio, hetarylthio, acyl, aroyl, hetaroyl, acyloxy, aroyloxy, hetaroyloxy, alkoxycarbonyl, aryloxycarbonyl, hetaryloxycarbonyl, alkylsulfonyl, fluorine, chlorine, bromine, iodine, cyano, nitro, sulfo, carbonyl, carboxy, carbamoyl, sulfamoyl,
it being possible for two adjacent moieties R to be linked to one another by means of an alkylene or alkenylene chain such as to form a 5-6-limbed, possibly aromatic ring system which if appropriate can contain one or more heteroatoms such as oxygen, nitrogen or sulphur,
it being possible, if appropriate, to substitute the moieties R¹-R⁴, themselves and independently of one another, for one or more of the aforementioned groups and by amino, alkylamino, dialkylamino, hydroxy, mercapto,
and salts, tautomers and metal complexes of compounds with the general formula (I) which have a urease-inhibiting effect.

2. The *N-*(2-nitrophenyl)phosphoric acid triamide according to Claim 1, in formula (I) X = O and R¹ = R² = R³ = R⁴ = H.

3. A process for the production of the *N-*(2-nitrophenyl)phosphoric acid triamides according to either Claim 1 or 2, **characterised in that**
a₁) one converts 2-nitroanilines or the hydrochlorides of the latter with phosphoryl chloride POCl₃) or thiphosphoryl chloride (PSCl₃), if appropriate in the presence of an organic solvent and a tertiary base, at temperatures of 0 to 150°C, optionally in an inert gas atmosphere, in accordance with equation (1) into type (A) *N*-(2-nitrophenyl)phosphoric acid amide chlorides, it also alternatively being possible to obtain compounds (A) with X = S by sulphurisation of the corresponding oxygen derivatives, 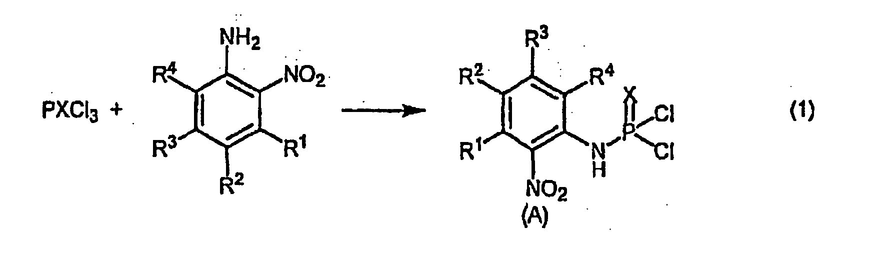 or
a₂) one allows phosphorpentachloride (PCl₅) to react with a 2-nitroaniline in an approximately equimolar ratio, optionally in an inert organic solvent, and optionally in an inert gas atmosphere at 0 to 150° C in accordance with equation (2) such as to form type (B) compounds which, optionally without any further isolation, are converted with an approximately equimolar quantity of formic acid or water into type (A) *N-*(2-nitrophenyl)phosphoric acid amide dichlorides, it being possible to obtain compounds (A) with X = S by sulfurisation of the corresponding oxygen derivatives, 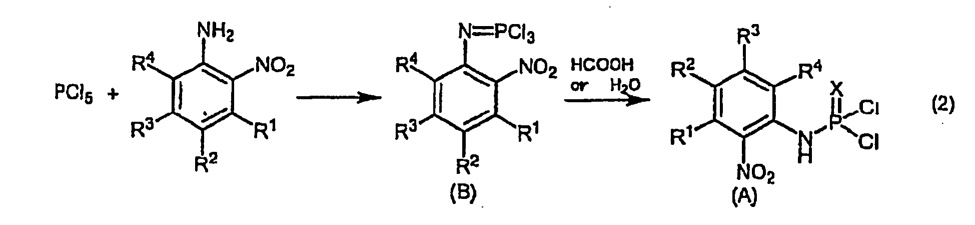 and then
b) one allows the type (A) compounds formed in step a₁) or a₂) to react with ammonia, optionally in an inert organic solvent, at temperatures of - 80 to 30°C in accordance with equation (3) to form the desired end product:

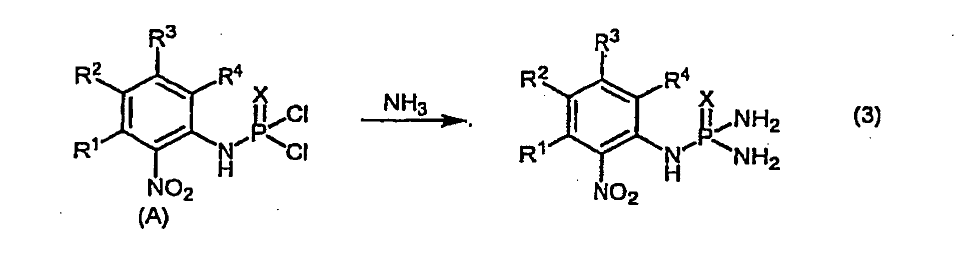

4. A composition, **characterised in that** it contains at least one *N-*(2-nitrophenyl)phosphoric acid triamide according to either of Claims 1 or 2 in a quantity sufficient to inhibit urease and optionally agriculturally and/or phsyiologically acceptable or compatible or desired carrier means, extenders or thinning agents, additives and optionally further active agents.

5. The composition according to Claim 4, **characterised in that** as a further active agent at least one nitrification inhibitor is included in a quantity effective for nitrification inhibition.

6. The composition according to Claim 5, **characterised in that** the nitrification inhibitor is selected from one or more of the following compounds:
a) pyrazole derivatives with the general formula (IV), or salts or complex compounds of the latter: 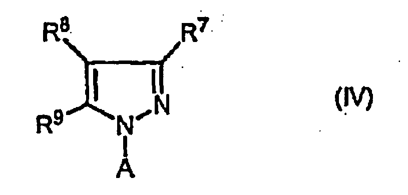 wherein
R⁷, R⁸, R⁹ mean, independently of one another, hydrogen, halogen, C₁-C₈-alkyl, or C₃-C₈-cycloalkyl
and
A means the moiety H
or the moiety 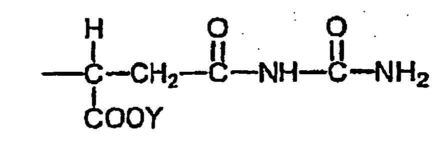 with Y = H, Na, K, NH₄
or the moiety
-CH₂-B with B = (di)alkylamino, 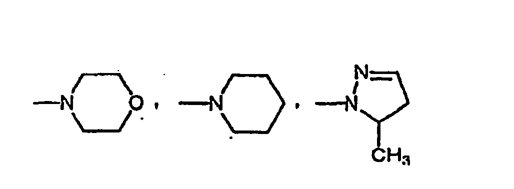 or the moiety 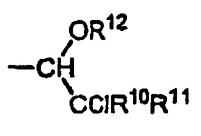 with R¹⁰, R¹¹ = H
or Cl and
R¹² = H or 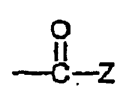 with Z = C₁-C₈-alkoxy, C₁-C₈-alkylamino, C₆-C₁₀-arylamino
or the moiety 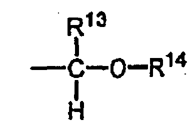 with R¹³, R¹⁴ = H, C₁-C₈-alkyl, C₇-C₁₈-alkylaryl, C₆-C₁₀-aryl or the moieties 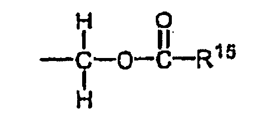 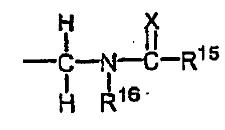 with R¹⁵ = C₁-C₂₀-alkyl, C₃-C₈-cycloalkyl, C₆-C₁₀-aryl or alkylaryl from C₁-C₄-alkyl and C₆-C₁₀-aryl groups: with X = oxygen or sulphur or with R¹⁶ = C₁-C₄-alkyl, C₃-C₈-cycloalkyl, C₆-C₁₀-aryl or H,
and the alkyl and aryl moieties listed can be substituted with themselves or with C₁-C₄-alkylsulfonyl, C₁-C₄-alkoxy, C₁-C₄-acyl, halogen, hydroxyl, trimethylsilyl, amino, nitro, sulfo, cyano, carbonyl, carboxyl or C₁-C₅-carboxylalkyl,
b) 1H-1,2,4-triazoles or salts or complex compounds of the latter,
c) dicyandiamide.

7. A fertiliser composition, **characterised in that** it contains at least one urea-based, mineral and/or organic fertiliser and at least one *N-*(2-nitrophenyl)phosphoric acid triamide according to either of Claims 1 or 2 and/or a composition according to any of Claims 4 to 6 or at least one *N-*(2-nitrophenyl)phosphoric acid triamide according to either of Claims 1 or 2 and at least one nitrification inhibitor defined in Claim 6 respectively in a quantity sufficient to inhibit urease or to inhibit nitrification.

8. The fertiliser composition according to Claim 7, **characterised in that** at least one *N-*(2-nitrophenyl)phosphoric acid triamide according to either of Claims 1 or 2 is included in a quantity of 0.001 to 10 % by weight in relation to the weight of the urea-based fertiliser.

9. The fertiliser composition according to Claim 7, **characterised in that** at least one of the nitrification inhibitors defined in Claim 6 is included in a quantity of 0.01 to 10 % by weight in relation to the weight of the urea-based fertiliser.

10. Use of the *N-*(2-nitrophenyl)phosphoric acid triamides according to either of Claims 1 or 2 or of the compositions according to any of Claims 4 to 6 for the extra-corporeal regulation or inhibition of the urease-catalysed urea hydrolysis.

11. Use of the *N-*(2-nitrophenyl)phosphoric acid triamides according to either of Claims 1 or 2 or of the compositions according to any of Claims 4 to 6 in order to reduce nitrogen losses when fertilising with fertilising urea or urea-based fertilisers.

12. Use of the *N-*(2-nitrophenyl)phosphoric acid triamides according to any of Claims 1 or 2 or of the compositions according to any of Claims 4 to 6 in order to reduce the ammonia contamination from the manure or from the animal excrement in animal sheds.

13. Use of *N-*(2-nitrophenyl)phosphoric acid triamides according to either of Claims 1 or 2 or of the compositions according to any of Claims 4 to 6 in order to avoid toxic effects when feeding feed urea within the framework of animal food.

14. Use of *N-*(2-nitrophenyl)phosphoric acid triamides according to either of Claims 1 or 2 or of the aqueous solutions or liquid formulations of the latter in order to stabilise urea-based fertilisers already produced or still to be produced by downstream or upstream application.

15. Use of the compositions according to any of Claims 4 to 6 for fertigation.

16. Use of *N-*(2-nitrophenyl)phosphoric acid triamides according to any of Claims 1 or 2 or of the composition according to Claim 4 in order to produce a drug for the prophylaxis or therapy of problems or diseases which were induced or promoted directly or indirectly by urease activity.

17. The use according to Claim 16, the problem or disease being selected from catheter incrustation, inflammatory and ulcerous gastric and intestinal diseases, urolithiasis, pyelonephritis, nephrolithiasis, ammonia encephalopathy, hepatic encephalopathy, hepatic coma, infections of the urinary passage and gastrointestinal infections.

18. The use according to Claim 17, the gastrointestinal infection being caused by Helicobacter pylori.

## Revendications

1. Triamides de l'acide *N-*(2-nitrophényl)phosphorique répondant à la formule générale (I) : 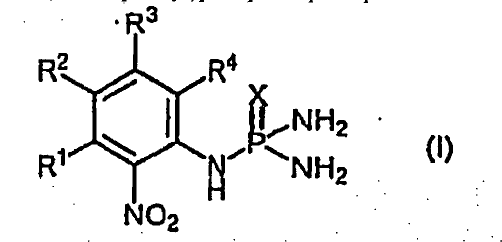 dans laquelle :
X désigne un atome d'oxygène ou de soufre
R¹, R², R³, R⁴ désignent, indépendamment les uns des autres, un atome d'hydrogène, un groupe alkyle/hétéroalkyle en C₁-C₈, alcényle/hétéroalcényle en C₂-C₈, alcinyle/hétéroalcinyle en C₂-C₈, cycloalkyle/hétérocycloalkyle en C₃-C₈, cycloalcényle/hétérocycloalcényle en C₃-C₈, aryle en C₆-C₁₀/hétéroaryle en C₅-C₁₀, aralkyle, hétéroarylalkyle, alkaryle, alkhétéroaryle, alcoxy, aryloxy, hétaryloxy, alkylthio, arylthio, hétarylthio, acyle, aroyle, hétaroyle, acyloxy, aroyloxy, hétaroyloxy, alcoxycarbonyle, aryloxycarbonyle, hétaryloxycarbonyle, alkylsulfonyle, un atome de fluor, chlore, brome, iode, un groupe cyano, nitro, sulfo, carbonyle, carboxy, carbamoyle, sulfamoyle,
deux radicaux R voisins pouvant être reliés l'un à l'autre par une chaîne alkylène ou alkénylène en formant, le cas échéant, un système cyclique de 5 à 6 chaînons ou un système cyclique aromatique pouvant comportant, le cas échéant, un ou plusieurs hétéroatomes tels que l'oxygène, l'azote ou le soufre,
les radicaux R¹ à R⁴ pouvant eux-mêmes, le cas échéant et indépendamment les uns des autres, être substitués par un ou plusieurs des groupes susmentionnés ainsi que par des groupes amino, alkylamino, dialkylamino, hydroxy, et mercapto,
ainsi que les sels, tautomères et complexes métalliques des composés répondant à la formule générale (I) ayant un effet inhibiteur de l'uréase.

2. Triamide de l'acide *N-*(2-nitrophényl)phosphorique selon la revendication 1, dans lequel, dans la formule (I), X représente un atome d'oxygène et R¹, R², R³ et R⁴ représentent chacun un atome d'hydrogène.

3. Procédé de production des triamides de l'acide *N-*(2-nitrophényl)phosphorique selon l'une des revendications 1 ou 2, **caractérisé en ce que**
a₁) on fait réagir des 2-nitroanilines ou leurs hydrochlorures avec du chlorure de phosphoryle (POCl₃) ou du chlorure de thiophosphoryle (PSCl₃), le cas échéant en présence d'un solvant organique et d'une base tertiaire, à des températures allant de 0 à 150 °C, le cas échéant sous atmosphère protectrice, selon l'équation (1) pour obtenir des dichlorures d'amide de l'acide *N-*(2-nitrophényl)phosphorique du type (A), des composés (A) dans lesquels X désigne le soufre pouvant être obtenus, en variante, par sulfuration des dérivés oxygénés correspondants, 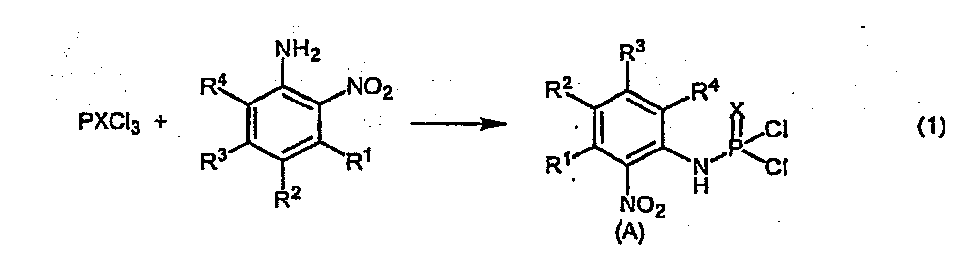 ou :
a₂) on fait réagir du pentachlorure de phosphore (PCl₅) avec une 2-nitroaniline, dans un rapport approximativement équimolaire, le cas échéant dans un solvant organique et le cas échéant sous atmosphère protectrice à 0 à 150 °C, selon l'équation (2) pour obtenir des composés du type (B), qui peuvent être mis à réagir, le cas échéant sans isolement supplémentaire, avec une quantité approximativement équimolaire d'acide formique ou d'eau pour obtenir des dichlorures d'amide de l'acide *N-*(2-nitrophényl)phosphorique du type (A), des composés (A) dans lesquels X désigne le soufre pouvant être obtenus par sulfuration des dérivés oxygénés correspondants, 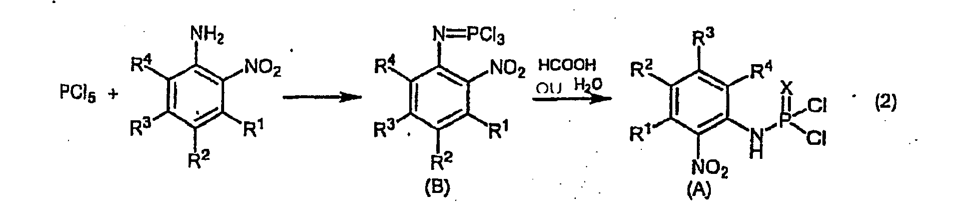 puis
b) on fait réagir les composés du type (A) formés dans l'étape a₁) ou a₂) avec de l'ammoniaque, le cas échéant dans un solvant organique inerte, à des températures allant de - 80 à 30 °C, selon l'équation (3) pour obtenir le produit final souhaité :

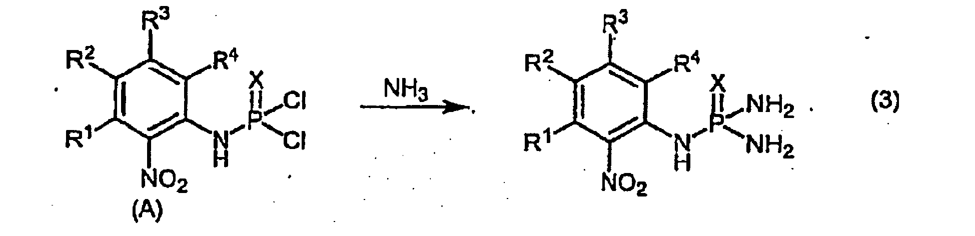

4. Composition, **caractérisée en ce qu'**elle contient au moins un triamide de l'acide *N-*(2-nitrophényl)phosphorique selon l'une des revendications 1 ou 2 dans une quantité suffisante pour inhiber l'uréase ainsi, le cas échéant, que des matières de support, des agents d'extension, des diluants, des adjuvants et, le cas échéant, d'autres agents actifs acceptables ou compatibles ou souhaités sur le plan agricole et/ou physiologique.

5. Composition selon la revendication 4, **caractérisée en ce que** l'autre agent actif contenu consiste au moins en un inhibiteur de nitrification dans une quantité efficace pour permettre l'inhibition de la nitrification.

6. Composition selon la revendication 5, **caractérisée en ce que** l'inhibiteur de nitrification est choisi parmi un ou plusieurs des composés suivants :
a) les dérivés du pyrazole répondant à la formule générale (IV), ou leurs sels ou composés complexes : 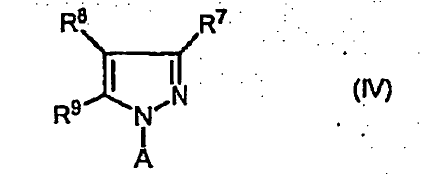 dans laquelle
R⁷, R⁸, R⁹, indépendamment les uns des autres, représentent un atome d'hydrogène, un halogène, un groupe alkyle en C₁-C₈, ou cycloalkyle en C₃-C₈
et
A représente le radical H
ou le radical 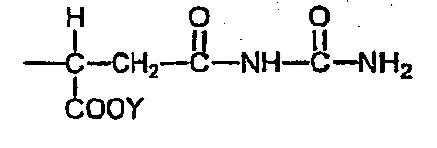 dans lequel Y = H, Na, K, NH₄
ou le radical
-CH₂-B dans lequel B = (di)alkylamino, 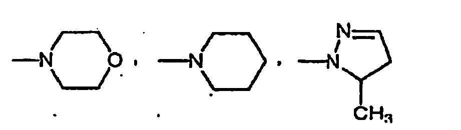 ou le radical 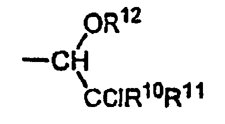 dans lequel R¹⁰, R¹¹ = H
ou Cl et
R¹²=H ou 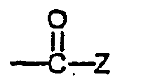 où Z = alcoxy en C₁-C₈, alkylamino en C₁-C₈, arylamino en C₆-C₁₀
ou le radical 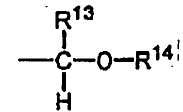 dans lequel R¹³, R¹⁴ = H, alkyle en C₁-C₈, alkylaryle en C₇-C₁₈, aryle en C₆-C₁₀
ou les radicaux 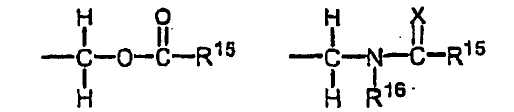 dans lesquels R¹⁵ représente un groupe alkyle en C₁-C₂₀, cycloalkyle en C₃-C₈, aryle en C₆-C₁₀ ou alkylaryle issu de groupes alkyle en C₁-C₄ et aryle en C₆-C₁₀ ; dans lesquels X représente un atome d'oxygène ou de soufre et dans lesquels R¹⁶ représente un groupe alkyle en C₁-C₄, cycloalkyle en C₃-C₈, aryle en C₆-C₁₀ ou un atome d'hydrogène,
et les radicaux alkyle et aryle indiqués pouvant être substitués par eux-mêmes ainsi que par des groupes alkylsulfonyle en C₁-C₄, alcoxy en C₁-C₄, acyle en C₁-C₄, halogéno, hydroxyle, triméthylsilyle, amino, nitro, sulfo, cyano, carbonyle, carboxyle ou carboxyalkyle en C₁-C₅,
b) les 1H-1,2,4-triazoles, ou leurs sels ou composés complexes,
c) le dicyandiamide.

7. Composition d'engrais, **caractérisée en ce qu'**elle contient au moins un engrais organique et/ou minéral à base d'urée ainsi au moins qu'un triamide de l'acide *N-*(2-nitrophényl)phosphorique selon l'une des revendications 1 ou 2 et/ou une composition selon l'une des revendications 4 à 6 ou au moins un triamide de l'acide *N-*(2-nitrophényl)phosphorique selon l'une des revendications 1 ou 2 et au moins un inhibiteur de nitrification défini dans la revendication 6 respectivement présent dans une quantité suffisante pour inhiber l'uréase ou la nitrification.

8. Composition d'engrais selon la revendication 7, **caractérisée en ce qu'**elle contient au moins un triamide de l'acide *N-*(2-nitrophényl)phosphorique selon l'une des revendications 1
ou 2 dans une quantité allant de 0,001 à 10 % en poids par rapport au poids de l'engrais à base d'urée.

9. Composition d'engrais selon la revendication 7, **caractérisée en ce qu'**elle contient au moins un des inhibiteurs de nitrification définis dans la revendication 6 dans une quantité allant de 0,01 à 10 % en poids, par rapport au poids de l'engrais à base d'urée.

10. Utilisation des triamides de l'acide *N-*(2-nitrophényl)phosphorique selon l'une des revendications 1 ou 2 ou des compositions selon l'une des revendications 4 à 6 pour la régulation ou l'inhibition extracorporelle de l'hydrolyse de l'urée catalysée par l'uréase.

11. Utilisation des triamides de l'acide *N-*(2-nitrophényl)phosphorique selon l'une des revendications 1 ou 2 ou des compositions selon l'une des revendications 4 à 6 pour réduire les déperditions d'azote lors de la fertilisation par apport d'engrais urée ou d'engrais à base d'urée.

12. Utilisation des triamides de l'acide *N-*(2-nitrophényl)phosphorique selon l'une des revendications 1 ou 2 ou des compositions selon l'une des revendications 4 à 6 pour réduire la pollution due à l'ammoniaque issue du lisier ou des excréments animaux dans les étables.

13. Utilisation des triamides de l'acide *N-*(2-nitrophényl)phosphorique selon l'une des revendications 1 ou 2 ou des compositions selon l'une des revendications 4 à 6 pour prévenir les effets toxiques dans le cas d'un apport d'urée alimentaire dans le cadre de l'alimentation des animaux.

14. Utilisation des triamides de l'acide *N-*(2-nitrophényl)phosphorique selon l'une des revendications 1 ou 2 ou de leurs solutions aqueuses ou formulations liquides pour stabiliser les engrais à base d'urée déjà épandus ou destinés à être épandus par application en amont ou en aval.

15. Utilisation des compositions selon l'une des revendications 4 à 6 pour la fertigation.

16. Utilisation des triamides de l'acide *N-*(2-nitrophényl)phosphorique selon l'une des revendications 1 ou 2 ou de la composition selon la revendication 4 pour la préparation d'un médicament destiné à la prévention ou à la thérapie des troubles ou maladies induites ou favorisées directement ou indirectement par l'activité uréase.

17. Utilisation selon la revendication 16, dans laquelle le trouble ou la maladie est choisie parmi l'encrassement des cathéters, les maladies inflammatoires et ulcéreuses de l'estomac et des intestins, l'urolithiase, la pyélonéphrite, la néphrolithiase, l'encéphalopathie ammoniacale, l'encéphalopathie hépatique, le coma hépatique, les infections des voies urinaires et les infections gastro-intestinales.

18. Utilisation selon la revendication 17, dans le cas où l'infection gastro-intestinale est due à *Helicobacter pylori.*
